# EUROPEAN PATENT SPECIFICATION

(11) **EP 3 363 470 B1**
(45) Date of publication and mention of the grant of the patent: **09.06.2021**
(21) Application number: 16855486.3
(22) Date of filing: 13.10.2016
(51) Int. Cl.: A61L 2/14, A61L 2/20, B65B 55/10, H05H 1/24, H05H 1/30, A61L 101/22, A61L 101/36

(54) **STERILIZATION APPARATUS AND METHOD**
STERILISATIONSVORRICHTUNG UND -VERFAHREN
UN DISPOSITIF DE STÉRILISATION ET UNE METHODE ASSOCIEE

(30) Priority: 13.10.2015 JP 2015202312; 13.10.2015 JP 2015202315
(43) Date of publication of application: 22.08.2018
(73) Proprietor: Suntory Holdings Limited, Osaka-shi, Osaka 530-8203 (JP)
(72) Inventor: HIGASHIYAMA, Kenichi, Kyoto 619-0284 (JP); TOMINAGA, Kenta, Kyoto 619-0284 (JP); HIRAYAMA, Yuji, Kyoto 619-0284 (JP); YOSHIHARA, Kazuki, Kyoto 619-0284 (JP)
(74) Representative: Mewburn Ellis LLP
(86) International application number: PCT/JP2016/080429
(87) International publication number: WO 2017/065233

(56) References cited:
- EP-A1- 1 736 175
- WO-A1-2007/071720
- WO-A1-2013/129473
- JP-A- H04 231 053
- JP-A- 2001 514 531
- JP-A- 2002 538 896
- JP-A- 2010 264 059
- JP-A- 2012 202 637
- KR-B1- 101 534 817
- US-A- 5 413 758

## Description

### TECHNICAL FIELD

The present invention relates to a sterilization method. More particularly, the present invention relates to a sterilization method in which sterilization treatment including irradiating reactive oxygen is carried out, and a sterilization apparatus for carrying out the sterilization method.

### BACKGROUND ART

Containers for foods or beverages (foodstuff) or the like are required to be sterilized on internal and external sides thereof. Conventionally, a method of sterilizing with a sterilizing agent has been widely used, and a method of spraying a mist containing a sterilizing agent to be vaporized, a method of blowing a mixture of a sterilizing agent with the air or the like have been known. In addition, as the sterilizing agent, hydrogen peroxide, a peracetic acid-based sterilizing agent such as peracetic acid, or a hypochlorous acid-based sterilizing agent such as an aqueous hypochlorous acid have been known.

However, some treatments after use are needed for the sterilizing agent, and further sterilization methods have been studied because if a high-concentration sterilizing agent is used to increase sterilization ability, the treatments therefor become costly, and the residual amount of the sterilizing agent becomes higher, thereby making them disadvantageous in the aspect of food sanitation.

For example, Patent Publication 1 has reported a sterilization method having excellent sterilization effects while reducing residual hydrogen peroxide by sterilizing a bottle for drink with a mist of hydrogen peroxide and air-rinsing with sterile air in view that when the mist of a sterilizing agent such as hydrogen peroxide is sprayed towards a bottle, this mist is adhered to various equipment of the apparatus, which are likely to corrode or damage them.

Patent Publication 2 discloses a sterilization method including preheating a chamber including dispersing heating media heated to a temperature higher than a sterilizing agent used in sterilization within a chamber; carrying out sterilization within the chamber including dispersing the sterilizing agent within a preheated chamber; and washing off the sterilizing agent including dispersing sterile water within the chamber. As described above, the patent publication describes that the temperature lowering of the sterilizing agent when dispersing the sterilizing agent can be controlled by preheating within the chamber prior to the dispersing of the sterilizing agent, so that the dispersion of the sterilizing agent is carried out within a temperature range that exhibits high sterilization ability.

On the other hand, Patent Publication 3 discloses a method including generating a plasma jet using discharge in a fluid, contacting a surface of an object with the plasma jet, and carrying out sterilization (disinfection) by way of energy transfer from the plasma jet to the surface. The plasma jet used in this publication is generated by atmospheric electric discharge in a process gas containing oxygen, preferably the air. Since this method exhibits some effects that sterilization is carried out with a fluid, so that not only smooth surfaces but also three-dimensional structures which are less likely to be reachable can be disinfected, contrary to a conventional sterilizing agent, whereby the residues do not remain at edges or corners.

EP05727568 discloses a sterilization chamber comprising a H₂O₂ inlet and a plasma generator. Plasma is generated under H₂O₂ atmosphere, directly in the chamber, which forms reactive oxygen species. There is no teaching of forming the reactive oxygen species before injection into the sterilization chamber filled with H₂O₂.

### RELATED ART REFERENCES

### PATENT PUBLICATIONS

Patent Publication 1: Japanese Patent Laid-Open No. 2014-15265
Patent Publication 2: Japanese Patent Laid-Open No. 2010-189034
Patent Publication 3: Japanese Unexamined Patent Publication No. 2009-519799

### SUMMARY OF THE INVENTION

### PROBLEMS TO BE SOLVED BY THE INVENTION

However, when a sterilizing agent is used, resistant bacteria have been found, and further improvements in sterilizing ability have been desired. However, the residual sterilizing agent with the improvements is still disadvantageous.

In addition, in the sterilization method of Patent Publication 3, the patent publication merely discloses a method of mixing a disinfectant substance in the form of a liquid in the process gas of the source from which plasma jet is generated in order to enhance the effects (see, [0025] of Patent Publication 3). It is unclear about the conditions that influence the plasma jet itself, and further techniques have been desired such that the residual sterilizing agents become bottleneck of the issues.

An object of the present invention is to provide a sterilization method having excellent sterilization effects and a sterilization apparatus for carrying out the sterilization method.

### MEANS TO SOLVE THE PROBLEMS

The present invention relates to
[1] A sterilization method according to present claim 6;
[2] A sterilization apparatus according to present claim 1.

### EFFECTS OF THE INVENTION

The sterilization method of the present invention exhibits some excellent effects that the sterilization effects are excellent. Also, a chemical or the like which has been used in conventional sterilization does not remain because the sterilization is carried out with a fluid, which leads to simplifications of processing steps, whereby productivity can be remarkably improved.

### BRIEF DESCRIPTION OF THE DRAWINGS

[FIG. 1] FIG. 1 is a schematic view showing one embodiment of a sterilization apparatus used in the present invention.
[FIG. 2] FIG. 2 is a schematic view showing one embodiment of a sterilization apparatus used in the present invention.
[FIG. 3] FIG. 3 is a view showing the inoculated sites to a resin bottle cap used in each of Test Examples.

### MODES FOR CARRYING OUT THE INVENTION

In general, reactive oxygen species (ROS) such as superoxide radicals (•O₂⁻), hydrogen peroxide (H₂O₂), or hydroxy radicals (HO•) exhibit an excellent sterilization action due to their strong oxidizing actions, and these are produced mainly from oxygen molecules or water in the air, and exhibit an excellent sterilization action by contacting with a surface of an object to be sterilized. Specifically, for example, hydroxy radicals are obtainable by reacting water molecules with plasma electrons. By using the reactive oxygen, the present invention has the feature that irradiation is carried out under the conditions that satisfy (A) in the presence of an oxidizing gas, and/or (B) under an environment of from 50 to 100% relative humidity. Hereinafter, a sterilization method under conditions satisfying (A) will be referred to as an embodiment A, and a sterilization method under conditions satisfying (B) will be referred to as an embodiment B. Here, in the present invention, the term "sterile" or "sterilization" means breaking of live bodies of microbes or removal thereof from surfaces to be sterilized, which, for example, includes disinfection, sterilization or sterile filtration.

### Embodiment A

Although the mechanisms by which the sterilization method of the embodiment A shows remarkably increased sterilization action as compared to a case where irradiation is carried out in the air are not elucidated in detail, they are assumed to be as follows. Since the reactive oxygen is contacted with an oxidizing gas in the course of moving to a surface of an object to be sterilized to react, the oxidizing gas is decomposed into hydroxy radicals having strongly oxidative strength to form hydroxy radical in chain reaction, thereby increasing an amount of reaction oxygen reaching the surface of an object to be sterilized. However, these assumptions do not limit the present invention thereto.

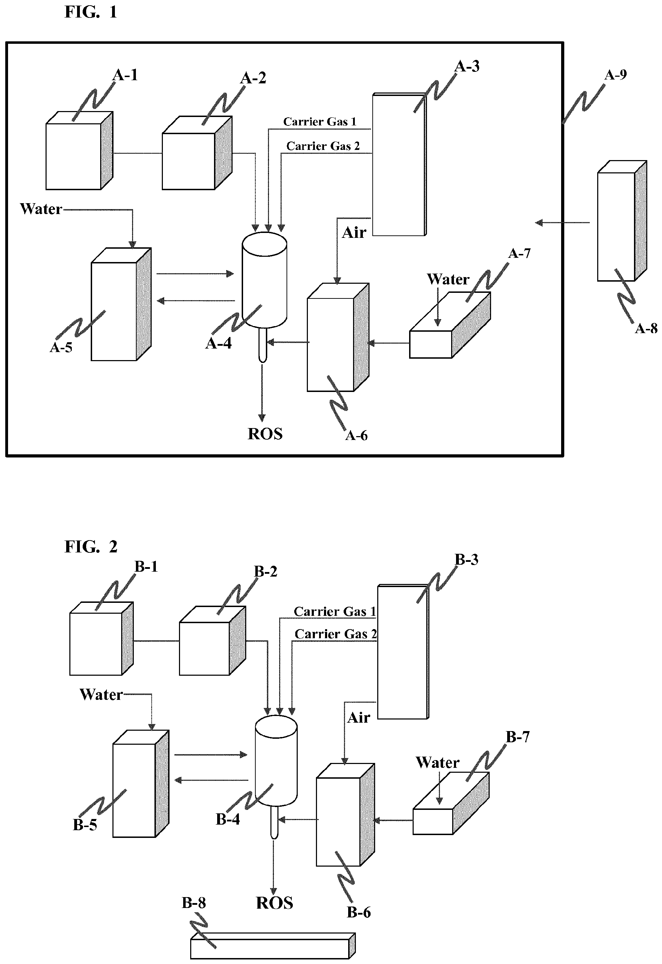

Sterilization apparatus usable in embodiment A will be explained in detail hereinbelow on the basis of FIG. 1. In embodiment A the reactive oxygen is not particularly limited, and those which are obtained by generating plasma using an alternating current and generating reactive oxygen from the plasma obtained will be explained. The sterilization apparatus shown in FIG. 1 is merely one embodiment of embodiment A, without intending to limit the present invention thereto.

As shown in FIG. 1, the sterilization apparatus usable in the embodiment A comprises each of the following units: an inlet unit A-1 for alternating current, a high-voltage unit A-2, an inlet unit A-3 for gas flow, a nozzle A-4, a chilling unit A-5 of the nozzle, an inlet unit A-6 for steam flow to the nozzle, an inlet unit A-7 for water flow to the inlet unit for steam flow, and an inlet unit A-8 for oxidizing gas flow, and a chamber A-9.

The inlet unit A-1 for alternating current is a source of generating electric charges of plasma discharge. The alternating current to be supplied is not particularly limited, and the alternating current includes, for example, ones generated at a frequency of from 10 to 15 kHz, and a voltage of from 200 to 500 V or so, which can be properly set in accordance with known techniques. The level of amperes of the alternating current is not particularly limited, and the level can be properly adjusted depending upon the specifications of the inlet device; for example, an alternating current of 11 A may be used. In embodiment A a direct current can be used in place of the alternating current, but alternating current is preferred, from the viewpoint of adjusting voltage.

The high-voltage unit A-2 is a device which is connected with the inlet unit A-1 for alternating current, and increases voltage of the alternating current supplied from the unit A-1, and any devices that are capable of increasing voltage can be used without particular problems. In addition, the high-voltage unit may be integrated with the unit A-1. The increased voltage is not particularly limited, and may be, for example, from 10 to 30 kV or so.

The inlet unit A-3 for gas flow is a device acting as inlet of gas flows of various gases to each of a nozzle A-4 and an inlet unit A-6 for steam flow, and a known inlet device for gas flow can be used.

Specifically, a carrier gas for generating plasma is allowed to flow to a nozzle A-4. As the carrier gas, the air, oxygen, nitrogen, argon, helium, and mixtures thereof can be used, among which it is preferable to use two kinds of the air and oxygen. The flow rate of the carrier gas is not unconditionally set, depending upon the size, shape, or the like of the nozzle A-4. For example, an embodiment includes allowing the air to flow at a rate of 6 L/min, and oxygen to flow at a rate of 3 L/min.

The air for mixing with the steam needed when producing reactive oxygen from plasma is allowed to flow to the inlet unit A-6 for steam flow. By using a water-containing gas in which the steam is mixed with the air, the mixing of the plasma and steam is accelerated, whereby hydroxy radicals can be efficiently produced from the steam. The flow rate of the air to the inlet unit A-6 for steam flow is the same as the flow rate of the water-containing gas to the nozzle A-4. For example, an embodiment of allowing the air to flow at a rate of 3 L/min is exemplified. Here, the air as used herein refers to a gas of which relative humidity is from 0 to 10% by volume or so at 20°C.

The nozzle A-4 is a device of irradiating reactive oxygen obtained by generating plasma, which is also referred to as a reactive oxygen irradiation unit. The device comprises an internal electrode and an external electrode, and an increased voltage is applied between both the electrodes from the high-voltage unit A-2, whereby making it possible to generate an electric field. In addition, the internal electrode may be connected with a coil, so that an even larger electric field can be formed. The shape, size or the like of the coil can be adjusted in accordance with the technical common knowledge of one of ordinary skill in the art.

In addition, the device comprises a gas inlet port and a reactive oxygen irradiation port, wherein the gas inlet port exists at an end of an opposite side to an end part at which the reactive oxygen irradiation inlet exists. Moreover, the gas inlet port is connected with a pipe from the inlet unit A-3 for gas flow, wherein plasma is generated by passing a carrier gas through the electric field generated as mentioned above. Since the plasma produced as described above is also a fluid, the plasma may also be referred to as a plasma jet. On the other hand, the reactive oxygen irradiation port has a tubular structure or a conical structure that is tapered toward a discharge opening, and connected with a pipe for allowing a water-containing gas to flow from the inlet unit A-6 for steam flow at any of the parts before reaching the discharge opening, at which a reactive oxygen would be produced by a reaction with the plasma produced above, and irradiated from the discharge opening of the reactive oxygen irradiation port.

The nozzle A-4 is not particularly limited in the shape or size so long as the nozzle has the above parts. For example, a structure comprising a gas inlet port arranged at an upper end of a cylindrical structure, and a reactive oxygen irradiation port having a tubular structure having a diameter smaller than the diameter of the apparatus at a lower end thereof is exemplified. The cylindrical structure may form a layered structure, and, for example, a structure in which a coil is formed in the surroundings of the tube through which a carrier gas passes, and optionally a layer of an insulation material is further formed in the surroundings of the coil is exemplified. The tube is not particularly limited so long as the tube is an electroconductive material, and known materials in the art can be used. In addition, the insulation material is not particularly limited, and a known insulation material in the art can be used.

The chilling unit A-5 of the nozzle is a device for allowing a chilling water to flow to the nozzle A-4, and a known device for chilling water flow can be used. Since the nozzle A-4 generates heat by applying a high voltage, it is preferable to chill the nozzle. As the chilling water, waters at temperatures of, for example, 5°C or so are preferably used, and the chilling water may be circulated between the nozzle A-4 and the chilling unit A-5. The flow rate of the chilling water can be properly adjusted so that the surface temperature of the nozzle A-4 is controlled to 25°C or lower. Here, the surface temperature of the nozzle A-4 can be measured with a contact-type thermometer.

The inlet unit A-6 for steam flow to the nozzle is a device of allowing a water-containing gas to flow to the nozzle A-4, and the inlet unit is connected to a reactive oxygen irradiation port of the nozzle A-4 as mentioned above. When the water-containing gas is allowed to flow, first, water from an inlet unit A-7 for water flow is heated with electric heating wires installed therein to produce steam, and a mixture of the steam with the air allowed to flow from the inlet unit A-3 for gas flow is allowed to flow to the nozzle A-4 as a water-containing gas. Here, the inlet unit A-7 for water flow may be integrated with the inlet unit A-6 for steam flow. The heating temperature of the electric heating wires can be properly adjusted depending upon the flow rate of water, which is exemplified by, for example, 300°C. Also, the flow rate of water from the inlet unit A-7 for water flow can be adjusted depending upon the amount of steam needed to produce reactive oxygen. In the embodiment A, the flow rate of water is preferably 0.5 mL/min or more, and more preferably 1.0 mL/min or more, from the viewpoint of containing a water content in the reactive oxygen-containing gas in an amount equal to or greater than the saturated steam. In addition, although the upper limit is not particularly set, the flow rate of water is preferably 6 mL/min or less, and more preferably 5 mL/min or less. The steam thus obtained is mixed with the air allowed to flow from the inlet unit A-3 for gas flow in a volume ratio (steam/the air) of from 0.2 to 2.5 or so, and the water-containing air is allowed to flow to a reactive oxygen irradiation port of the nozzle A-4. The mixing volume ratio of steam to the air can be modified by fluctuating the flow rate of water as mentioned above, and the amount of steam contained in the water-containing air can be increased by increasing the flow rate of water. Examples of the mixing volume ratio of the plasma jet produced in the nozzle A-4 to the water-containing gas allowed to flow from the inlet unit A-6 for steam flow [plasma jet / water-containing gas] include from 0.8 to 2.6.

The inlet unit A-8 for oxidizing gas flow is a device of allowing an oxidizing gas to flow, and the inlet unit is not particularly limited so long as the unit is arranged so that the reactive oxygen from the nozzle A-4 is irradiated in the presence of an oxidizing gas. For example, reactive oxygen can be irradiated from a nozzle A-4 in the presence of an oxidizing gas so long as a shielding space is provided, and at least a nozzle A-4 is present therein, and an oxidizing gas is allowed to flow within the space. Therefore, the inlet unit A-8 for oxidizing gas flow and the nozzle A-4 do not exist in the same space. In addition, structurally, a known apparatus for gas flow can be used so long as an oxidizing gas is allowed to flow. The oxidizing gas usable in the present invention is, but not particularly limited to, preferably selected from hydrogen peroxide and peracetic acid, from the viewpoint of sterilization effects, and the oxidizing gas may be combined and used with other known gases so long as the gases are not reactive with each other. In addition, a liquid product containing a component of an oxidizing gas that is vaporized in accordance with a known technique may be allowed to flow. The oxidizing gas does not need to be allowed to flow at a given level so long as the oxidizing gas is present within the chamber while irradiating reactive oxygen, and the oxidizing gas may be intermittently allowed to flow beforehand. The flow rate from the inlet unit A-8 for oxidizing gas flow can be appropriately set.

The chamber A-9 is provided to form the above shielding space. The chamber A-9 may be an embodiment in which at least a nozzle A-4 of the above units is contained in its internal, and its size and structure can be appropriately set depending upon an object to be sterilized. The oxidizing gas is allowed to flow from the inlet unit A-8 for oxidizing gas flow in the chamber A-9, and the existing amount of the oxidizing gas is preferably 0.1 ppmv or more, and more preferably 1.5 ppmv or more, from the viewpoint of improving sterilization effects. The upper limit is not particularly set, and the existing amount is preferably 2 ppmv or less, from the viewpoint of safety. Although it would be difficult to sterilize an object to be sterilized with the amount of an oxidizing gas that exists within the chamber, in the present invention, remarkable sterilization effects are exhibited by irradiating reactive oxygen even in the presence of an oxidizing gas of that level. Here, the temperature within the chamber is not particularly set, and the temperature is, for example, from 2° to 40°C.

Here, the sterilization apparatus usable in embodiment A may further comprise other units besides the units mentioned above. Examples of other units include irradiation platforms on which objects to be sterilized are placed; shielding walls for preventing diffusion of reactive oxygen, and the like. The irradiation platform is not particularly limited so long as the object to be sterilized can be placed, and it is preferable that the object to be sterilized can be placed at a temperature equal to or lower than an ordinary temperature (40°C), from the viewpoint of not decomposing hydroxy radicals due to high temperatures.

The sterilization apparatus usable in embodiment A is not particularly limited so long as it has the specifications and constitutions mentioned above, and, a preferred example includes, for example, a sterilization apparatus comprising a reactive oxygen irradiation unit for irradiating reactive oxygen, and an inlet unit for oxidizing gas flow for allowing an oxidizing gas to flow so that a reactive oxygen from the reactive oxygen irradiation unit is irradiated to the object to be sterilized in the presence of an oxidizing gas.

Thus, a reactive oxygen is irradiated from the sterilization apparatus of the present invention. The reactive oxygen is irradiated in the presence of an oxidizing gas, so that the amount of hydroxy radicals formed becomes large, which in turn gives excellent sterilization activity. Also, since the reactive oxygen is a fluid, some excellent effects are exhibited in that even a three-dimensional structured object can be sterilized, so that residues do not remain in edges and corners.

### Embodiment B

Although the mechanisms by which the sterilization method of embodiment B shows remarkably increased sterilization action as compared to a case where irradiation is carried out in the air are not elucidated in detail, they are assumed to be as follows. When reactive oxygen is irradiated under the atmosphere of a relative humidity of 50% or more, and preferably 60% or more, and 100% or less, and preferably 90% or less, the steam contained in the environment is directly contacted with an object to be sterilized, a temperature is lowered upon the contact, which leads to the generation of dewfalls. The condensation contains reactive oxygen such as hydroxy radicals, and the present inventors have made studies in view that it is important to maintain dewfalls. As a result, the amount of dewfalls becomes large by irradiating reactive oxygen under a relative humidity as defined above, so that retention of reactive oxygen is increased, which in turn remarkably increases the sterilization action. However, these assumptions do not limit the present invention thereto. Here, in the present invention, relative humidity refers to a ratio of the water vapor pressure of the actual air relative to saturated water vapor pressure at the given temperature, expressed by "% (percent)."

The reactive oxygen in the embodiment B can be generated with plasma generated by a known method. For example, in the embodiment B, as an example of using plasma generated using an alternating current, an example of using a sterilization apparatus having the following constitution will be explained on the basis of FIG. 2. The sterilization apparatus shown in FIG. 2 is merely one embodiment of embodiment B, without intending to limit the present invention thereto.

As shown in FIG. 2, the sterilization apparatus usable in the embodiment B comprises each units of an inlet unit B-1 for alternating current, a high-voltage unit B-2, an inlet unit B-3 for gas flow, a nozzle B-4, a chilling unit B-5 of the nozzle, an inlet unit B-6 for steam flow to the nozzle, an inlet unit B-7 for water flow to the inlet unit for steam flow, and an irradiation platform B-8. The chamber and chamber inlet unit required in claim 1 are not shown.

The inlet unit B-1 for alternating current is a source of generating electric charges of plasma discharge. The alternating current to be supplied is not particularly limited, and the alternating current includes, for example, ones generated at a frequency of from 10 to 15 kHz, and a voltage of from 200 to 500 V or so, which can be properly set in accordance with known techniques. The level of amperes of the alternating current is not particularly limited, and the level can be properly adjusted depending upon the specifications of the inlet device; for example, an alternating current of 11 A may be used. In embodiment B, a direct current can be used in place of the alternating current, but the alternating current is preferred, from the viewpoint of adjusting voltage.

The high-voltage unit B-2 is a device which is connected with the inlet unit B-1 for alternating current, and increases voltage of the alternating current supplied from the unit B-1, and any of devices that are capable of increasing voltages can be used without particular problems. In addition, the high-voltage unit may be integrated with the unit B-1. The increased voltage is not particularly limited, and may be, for example, from 10 to 30 kV or so.

The inlet unit B-3 for gas flow is an device for inlet of gas flows of various gases to each of a nozzle B-4 and an inlet unit B-6 for steam flow, and a known inlet device for gas flow can be used.

Specifically, a carrier gas for generating plasma is allowed to flow to a nozzle B-4. As the carrier gas, the air, oxygen, nitrogen, argon, helium, and mixtures thereof can be used, among which it is preferable to use two kinds of the air and oxygen. The flow rate of the carrier gas is not unconditionally set, depending upon the size, shape, or the like of the nozzle B-4. For example, an embodiment includes allowing the air to flow at a rate of 6 L/min, and oxygen to flow at a rate of 3 L/min.

The air for mixing with the steam needed when producing reactive oxygen from plasma is allowed to flow to the inlet unit B-6 for steam flow. By using a water-containing gas in which the steam is mixed with the air, the mixing of the plasma and steam is accelerated, whereby hydroxy radicals can be efficiently produced from the steam. The flow rate of the air to the inlet unit B-6 for steam flow is the same as the flow rate of the water-containing gas to the nozzle B-4. For example, an embodiment of allowing the air to flow at a rate of 3 L/min is exemplified. Here, the air as used herein refers to a gas of which relative humidity is from 0 to 10% by volume or so at 20°C.

The nozzle B-4 is a device of irradiating reactive oxygen obtained by generating plasma, which is also referred to as a reactive oxygen irradiation unit. The device comprises an internal electrode and an external electrode, and an increased voltage is applied between both the electrodes from the high-voltage unit B-2, whereby making it possible to generate an electric field. In addition, the internal electrode may be connected with a coil, so that an even larger electric field can be formed. The shape, size or the like of the coil can be adjusted in accordance with the technical common knowledge of one of ordinary skill in the art.

In addition, the device comprises a gas inlet port and a reactive oxygen irradiation port, wherein the gas inlet port exists at an end of an opposite side to an end part at which the reactive oxygen irradiation inlet exists. Moreover, the gas inlet port is connected with a pipe from the inlet unit B-3 for gas flow, wherein plasma is generated by passing a carrier gas through the electric field generated as mentioned above. Since the plasma produced as described above is also a fluid, the plasma may also be referred to as a plasma jet. On the other hand, the reactive oxygen irradiation port has a tubular structure or a conical structure that is tapered toward a discharge opening, and connected with a pipe for allowing a water-containing gas to flow from the inlet unit B-6 for steam flow at any of the parts before reaching the discharge opening, at which a reactive oxygen would be produced by a reaction with the plasma produced above, and irradiated from the discharge opening of the reactive oxygen irradiation port.

The nozzle B-4 is not particularly limited in the shape or size so long as the nozzle has the above parts. For example, a structure comprising a gas inlet port arranged at an upper end of a cylindrical structure, and a reactive oxygen irradiation port having a tubular structure having a diameter smaller than the diameter of the apparatus at a lower end thereof is exemplified. The cylindrical structure may form a layered structure, and, for example, a structure in which a coil is formed in the surroundings of the tube through which a carrier gas passes, and optionally a layer of an insulation material is further formed in the surroundings of the coil is exemplified. The tube is not particularly limited so long as the tube is an electroconductive material, and known materials in the art can be used. In addition, the insulation material is not particularly limited, and a known insulation material in the art can be used.

The chilling unit B-5 of the nozzle is a device for allowing a chilling water to flow to the nozzle B-4, and a known device for chilling water flow can be used. Since the nozzle B-4 generates heat by applying a high voltage, it is preferable to chill the nozzle. As the chilling water, waters at temperatures of, for example, 5°C or so are preferably used, and the chilling water may be circulated between the nozzle B-4 and the chilling unit B-5. The flow rate of the chilling water can be properly adjusted so that the surface temperature of the nozzle B-4 is controlled to 25°C or lower. Here, the surface temperature of the nozzle B-4 can be measured with a contact-type thermometer.

The inlet unit B-6 for steam flow to the nozzle is a device of allowing a water-containing gas to flow to the nozzle B-4, and the inlet unit is connected to a reactive oxygen irradiation port of the nozzle B-4 as mentioned above. When the water-containing gas is allowed to flow, first, water from an inlet unit B-7 for water flow is heated with electric heating wires installed therein to produce steam, and a mixture of the steam with the air allowed to flow from the inlet unit B-3 for gas flow is allowed to flow to the nozzle B-4 as a water-containing gas. Here, the inlet unit B-7 for water flow may be integrated with the inlet unit B-6 for steam flow. The heating temperature of the electric heating wires can be properly adjusted depending upon the flow rate of water, which is exemplified by, for example, 300°C. Also, the flow rate of water from the inlet unit B-7 for water flow can be adjusted depending upon the amount of steam needed to produce reactive oxygen. In the embodiment B, the flow rate of water is preferably 0.5 mL/min or more, and more preferably 1.0 mL/min or more, from the viewpoint of containing a water content in the reactive oxygen-containing gas in an amount equal to or greater than the saturated steam. In addition, although the upper limit is not particularly set, the flow rate of water is preferably 6 mL/min or less, and more preferably 5 mL/min or less. The steam thus obtained is mixed with the air allowed to flow from the inlet unit B-3 for gas flow in a volume ratio (steam/the air) of from 0.2 to 2.5 or so, and the water-containing air is allowed to flow to a reactive oxygen irradiation port of the nozzle B-4. The mixing volume ratio of steam to the air can be modified by fluctuating the flow rate of water, and the amount of steam contained in the water-containing air can be increased by increasing the flow rate of water. Examples of the mixing volume ratio of the plasma jet produced in the nozzle B-4 to the water-containing gas allowed to flow from the inlet unit B-6 for steam flow [plasma jet / water-containing gas] include from 0.8 to 2.6.

The irradiation platform B-8 on which an object to be sterilized is placed is not particularly limited so long as the object to be sterilized can be placed, and it is preferable that the object to be sterilized can be placed at a temperature equal to or lower than an ordinary temperature (40°C), from the viewpoint not decomposing hydroxy radicals due to high temperatures.

Here, the sterilization apparatus usable in embodiment B may further comprise other units besides the units mentioned above. Examples of other units include shielding walls for preventing diffusion of reactive oxygen, and the like

In embodiment B the controlling method is not particularly limited, so long as the relative humidity of the atmosphere in which reactive oxygen is irradiated is 50% or more, and preferably 60% or more, and 100% or less, and preferably 90% or less. For example, the relative humidity inside the working room including internals including at least a nozzle B-4 and an irradiation platform B-8 out of the above units (and the features required in claim 1) can be
controlled using an air-conditioning facility such as air-conditioner. The temperature inside the working room is not particularly set, and the temperature is, for example, from 2° to 40°C. In addition, the sterilization apparatus usable in embodiment B itself may be set within a chamber or the like which is humidity-controllable.

Thus, reactive oxygen is irradiated under specified relative humidity conditions, so that hydroxy radicals are more retained, which in turn makes it possible to show excellent sterilization activity. Also, since reactive oxygen is a fluid, some excellent effects are exhibited that a three-dimensional structured object can be sterilized, so that residues do not remain on edges or corners.

### Embodiment C

In addition, one embodiment of the present invention includes an embodiment of carrying out irradiation using a reactive oxygen under the conditions satisfying (C) the environment of an absolute humidity of from 14.1 to 25.0 g/m³ (embodiment C). In the same manner as under the conditions of specified relative humidity in embodiment B, it is assumed that an amount of condensation generated on the surface of an object to be sterilized becomes large, so that the retention of the reactive oxygen is increased, which in turn remarkably increases the sterilization action. However, these assumptions do not limit the present invention thereto. Here, the absolute humidity in the present invention refers to a density of steam contained in the air (mass per volume), which is expressed as "g/m³."

In the sterilization apparatus usable in the embodiment C, the absolute humidity is not particularly limited, so long as the lower limit of the absolute humidity may be 14.1 g/m³ or more, and preferably 14.6 g/m³ or more, and the upper limit satisfies 25.0 g/m³ or less, preferably 20.0 g/m³ or less, and more preferably 18.0 g/m³ or less. For example, the sterilization apparatus usable in the embodiment B can be suitably used. The constitution and specifications of the apparatus, the methods of use, the conditions of use, and the like can be appropriately set referring to the embodiment B. The absolute humidity can be controlled in the same manner as controlling the relative humidity in the embodiment B.

In the present invention, the sterilization method is not particularly limited, so long as the sterilization method of embodiment A, embodiment B, or embodiment C mentioned above can be carried out, and these embodiments can be used alone or in a combination of two or more kinds. Specific examples of the combination include, for example, in a case of embodiment A and embodiment B, a method including carrying out sterilization under conditions that satisfy both of in the presence of an oxidizing gas and a relative humidity of from 50 to 100%. Also, in a case of embodiment A and embodiment C, specific methods include a method including carrying out sterilization under conditions that satisfy both of in the presence of an oxidizing gas and an absolute humidity of from 14.1 to 25.0 g/m³.

The reactive oxygen to be irradiated in the present invention is warm due to electric discharge within the nozzle or the water-contained gas from the inlet unit for steam flow, the temperature of which is from about 50° to about 80°C. Because of this warmth, the heated load of the irradiated object is considered to be small. Here, the temperature of the reactive oxygen refers to a temperature of reactive oxygen at a discharge opening of the reactive oxygen irradiation port that is measured with a thermocouple thermometer.

In addition, a temperature difference between the reactive oxygen and the surface of the object to be sterilized is, for example, preferably 10°C or more, and more preferably from 25° to 40°C, from the viewpoint of increasing a reactivity of radicals. The temperature of the surface of the object to be sterilized as used herein refers to a temperature of an object to be sterilized that is measured with a contact-type thermometer.

The irradiation speed can be adjusted according to the flow rate of the gas and the shape of the reactive oxygen irradiation port, and, for example, the irradiation speed includes 50,000 mm/sec. The irradiation time is not unconditionally set depending upon the object, and an irradiation time is exemplified by, for example, from 0.05 to 1 second.

In addition, it is preferable that the distance between the reactive oxygen irradiation port and the surface of the object to be sterilized is, for example, from 5 to 50 mm.

The sterilization method of the present invention is used for irradiating reactive oxygen to an object in need of sterilization. The object is exemplified by, for example, containers for foodstuff, bottle caps for sealing the opening part of the containers, medical devices, foodstuff such as vegetables and meat, and the like.

The present invention also provides a sterilization apparatus for irradiating a reactive oxygen. Here, the apparatus for irradiating a reactive oxygen includes the apparatuses suitably used in the sterilization method of the present invention.

Specifically, regarding a sterilization method of embodiment A, the sterilization apparatus includes a sterilization apparatus comprising a reactive oxygen irradiation unit for irradiating a reactive oxygen and an inlet unit for oxidizing gas flow which allows to flow an oxidizing gas so that the reactive oxygen from the reactive oxygen irradiation unit is irradiated to an object to be sterilized in the presence of the oxidizing gas. The generation conditions or specifications of reactive oxygen or an oxidizing gas, and a setting method are as prescribed in the section of the sterilization method of embodiment A.

Regarding a sterilization method of embodiment B, the sterilization apparatus includes a sterilization apparatus comprising a reactive oxygen irradiation unit for irradiating a reactive oxygen and a unit for adjusting humidity so that the reactive oxygen from the reactive oxygen irradiation unit is irradiated to an object to be sterilized under the environment of a relative humidity of from 50 to 100%. The generation conditions or specifications of reactive oxygen, a method for adjusting humidity, and a setting method are as prescribed in the section of the sterilization method of embodiment B.

Regarding a sterilization method of embodiment C, the sterilization apparatus includes a sterilization apparatus comprising a reactive oxygen irradiation unit for irradiating a reactive oxygen and a unit for adjusting a warm humidity so that the reactive oxygen from the reactive oxygen irradiation unit is irradiated to an object to be sterilized under the environment of an absolute humidity of from 14.1 to 25.0 g/m³, preferably from 14.1 to 20.0 g/m³, more preferably from 14.6 to 20.0 g/m³, and even more preferably from 14.6 to 18.0 g/m³. The generation conditions or specifications of reactive oxygen, a method for adjusting warm humidity, and a setting method are as prescribed in the section of the sterilization method of the embodiment C.

The sterilization apparatus of the present invention is used for irradiating reactive oxygen to an object in need of sterilization. The object is exemplified by, for example, containers for foodstuff, bottle caps for sealing the opening part of the containers, medical devices, foodstuff such as vegetables and meat, and the like.

### EXAMPLES

The present invention will be described more specifically by means of Examples given hereinbelow, without intending to limit the present invention thereto.

### TEST EXAMPLE A-1

The influences of the oxidizing gas on sterilization were studied.

### Preparation of Bacterial Solution and Preparation of Bacteria-Inoculated Bottle Cap

Using a bacterial solution of fibroblast bacterium *Bacillus atrophaeus,* bacterial solutions of various concentrations (3 standards within the concentration range of from 2 × 10³ to 2 × 10⁸ CFU/mL) were prepared. The obtained bacterial solution was inoculated in a resin bottle cap (material: polyethylene) as shown in FIG. 2 in an amount of 1 µL × 9 spots for each bottle cap (each concentration n = 5). Here, the inoculated resin bottle cap which was allowed to stand in a sterile petri dish for 24 hours to dryness was used.

### Irradiation of Reactive Oxygen

Using the sterilization apparatus shown in FIG. 1, a resin bottle cap that was inoculated was irradiated with reactive oxygen for 0.2 seconds per bottle cap from a distance 30 mm upstream side, and the irradiated bottle cap was collected on a sterile petri dish. Here, the operating conditions of the sterilization apparatus were as follows. When the oxidizing gas was allowed to flow from the inlet unit A-8 for oxidizing gas flow, a container containing 200 mL of peracetic acid was heated with stirring until boiling, the generated steam was introduced into a beaker separately set within a chamber A-9 through a tube, and added dropwise into the beaker, and the chamber A-9 was filled up with the oxidizing gas, after which a reactive oxygen was irradiated. Also, as to the sample not irradiated with reactive oxygen, the inoculated resin bottle cap was allowed to stand within a sterilization apparatus for 0.2 seconds in which the oxidizing gas was filled up. The surface temperature of the bottle cap (a surface temperature of the irradiation platform) was 25°C, and a temperature inside the chamber was 28°C. Here, the concentration of the oxidizing gas within the chamber was calculated by assuming that an amount filled within the chamber was an amount subtracting a liquid amount of remaining gas after dropping into the beaker from the gas liquid volume before use.

### Operating Conditions of Sterilization Apparatus

Inlet unit A-1 for alternating current: frequency: 14 kHz, voltage: 300 V, electric current: 11A
High-voltage unit A-2: The raised voltage: 20 kV
Inlet unit A-3 for gas flow: flow rate of air: 6 L/min, flow rate of oxygen: 3 L/min (hereinabove, go to nozzle A-4), flow rate of air: 3 L/min (go to inlet unit A-6 for steam flow)
Nozzle A-4: temperature of reactive oxygen irradiation: 51°C, irradiation speed: 50,000 mm/sec
Chilling unit A-5: chilling water: 5°C
Inlet unit A-6 for steam flow: electric heating wires: 300°C, flow rate of water-containing gas: 4.5 L/min (flow rates of plasma jet/water-containing gas flow (volume ratio) = 9/4.5)
Inlet unit A-7 for water flow: flow rate of water: 1.2 mL/min Chamber A-9: The concentration of an oxidizing gas shown in Table 1.

### Measurement of Sterilization Activity Values

A resin bottle cap subjected to irradiation of reactive oxygen or a resin bottle cap without subjection to irradiation and allowed to stand in a sterilization apparatus filled with an oxidizing gas was taken out of the sterile petri dish, and 5 mL of TSA liquid medium (manufactured by BD Falcon) was injected to the bottle cap, and cultured at 35°C, a temperature suitable for proliferation of microbes for 3 days. After the cultivation, the number of bottle caps in which media became turbid due to microbial proliferation that was judged as positive was counted, and the sterilization activity value LRV (Log Reduction Value) was calculated according to the most probable number method (MPN method). The results are shown in Table 1. Here, the "D" value showing the sterilization activity is expressed by common logarithm (LOG value) of the number of bacteria per bottle cap, which is a value obtained by subtracting the number of bacteria after the treatment (LOG value) from the number of bacteria before the treatment (LOG value). It is shown that the larger the number, the higher the sterilization activity, and the number of 4.5 D or more would show no problem as the sterilization treatment of food containers..

### Table 1

**Table 1**

| | Whether or not oxidizing agent was allowed to flow | Concentration of oxidizing gas (ppmv) | Whether or not reactive oxygen was irradiated | LRV (D Value) |
|---|---|---|---|---|
| Comp. Ex. A-1 | No | 0 | Yes | 4.5 D |
| Comp. Ex. A-2 | Yes | 1.6 | No | 1.1 D or less |
| Ex. A-1 | Yes | 1.6 | Yes | 5.1 D |

It is clear from Table 1 that although there are no sterilization effects found in the amount of oxidizing gas of Comparative Example A-2, from the comparisons of Comparative Example A-1 and Example A-1, Example A-1 in which the oxidizing gas is present when irradiating the reactive oxygen has remarkably improved sterilization effects. As described above, it is suggested that excellent sterilization effects are obtained by irradiating the reactive oxygen in the presence of an oxidizing gas. In addition, since the existing amount of the oxidizing gas is very small, the sterilizing agent does not remain, leading to simplification of steps, whereby productivity can be remarkably improved.

### TEST EXAMPLE B-1 (reference example)

The influences of the relative humidity on sterilization were studied.

### Preparation of Bacterial Solution and Preparation of Bacteria-Inoculated Bottle Cap

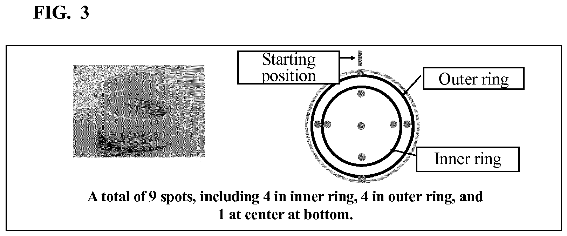

Using a bacterial solution of fibroblast bacterium *Bacillus atrophaeus,* bacterial solutions of various concentrations (3 standards within the concentration range of from 2 × 10³ to 2 × 10⁸ CFU/mL) were prepared. The obtained bacterial solution was inoculated in a resin bottle cap (material: polyethylene) as shown in FIG. 3 in an amount of 1 µL × 9 spots for each bottle cap (each concentration n = 5). Here, the inoculated resin bottle cap which was allowed to stand in a sterile petri dish for 24 hours to dryness was used.

### Irradiation of Reactive Oxygen

Using the sterilization apparatus shown in FIG. 2, a resin bottle cap that was inoculated was irradiated with reactive oxygen under the environment of a relative humidity as listed in Table 2, for 0.5 seconds per bottle cap from a distance 30 mm upstream side, and the irradiated bottle cap was collected on a sterile petri dish. Here, the operating conditions of the sterilization apparatus were as follows. The chamber environment in which the sterilization apparatus was set was controlled by an air-conditioning facility, and a setting humidity was set at a relative humidity as listed in Table 2.

### Operating Conditions of Sterilization Apparatus

Inlet unit B-1 for alternating current: frequency: 14 kHz, voltage: 300 V, electric current: 11A
High-voltage unit B-2: The raised voltage: 20 kV
Inlet unit B-3 for gas flow: flow rate of air: 6 L/min, flow rate of oxygen: 3 L/min (hereinabove, go to nozzle B-4), flow rate of air: 3 L/min (go to inlet unit B-6 for steam flow)
Nozzle B-4: temperature of reactive oxygen irradiation: 51°C, irradiation speed: 50,000 mm/sec
Chilling unit B-5: chilling water: 5°C
Inlet unit B-6 for steam flow: electric heating wires: 300°C, flow rate of water-containing gas: 4.5 L/min (flow rates of plasma jet/water-containing gas flow (volume ratio) = 9/4.5)
Inlet unit B-7 for water flow: flow rate of water: 1.2 mL/min

### Measurement of Sterilization Activity Values

A resin bottle cap was taken out of the sterile petri dish, and 5 mL of TSA liquid medium (manufactured by BD Falcon) was injected to the bottle cap, and cultured at 35°C, a temperature suitable for proliferation of microbes for 3 days. After the cultivation, the number of bottle caps in which media became turbid due to microbial proliferation that was judged as positive was counted, and the sterilization activity value LRV (Log Reduction Value) was calculated according to the most probable number method (MPN method). The results are shown in Table 2. Here, the "D" value showing the sterilization activity is expressed by common logarithm (LOG value) of the number of bacteria per bottle cap, which is a value obtained by subtracting the number of bacteria after the treatment (LOG value) from the number of bacteria before the treatment (LOG value). It is shown that the larger the number, the higher the sterilization activity, and the number of 4.5 D or more would show no problem as the sterilization treatment of food containers.

### Table 2

**Table 2**

| | Relative Humidity (%) | LRV (D Value) |
|---|---|---|
| Comp. Ex. B-1 | 40 | 5.2 D |
| Ex. B-1 | 65 | 6.0 D or More |

It is suggested from Table 2 that one having a higher relative humidity can give more excellent sterilization effects from the comparisons of Comparative Example B-1 and Example B-1.

### TEST EXAMPLE C-1 (reference example)

The influences of the absolute humidity on sterilization were studied.

### Preparation of Bacterial Solution and Preparation of Bacteria-Inoculated Bottle Cap

Using a bacterial solution of fibroblast bacterium *Bacillus atrophaeus,* bacterial solutions of various concentrations (3 standards within the concentration range of from 2 × 10³ to 2 × 10⁸ CFU/mL) were prepared. The obtained bacterial solution was inoculated in a resin bottle cap (material: polyethylene) as shown in FIG. 3 in an amount of 1 µL × 9 spots for each bottle cap (each concentration n = 5). Here, the inoculated resin bottle cap which was allowed to stand in a sterile petri dish for 24 hours to dryness was used.

### Irradiation of Reactive Oxygen

Using the sterilization apparatus shown in FIG. 2, a resin bottle cap that was inoculated was irradiated with reactive oxygen under the environment of an absolute humidity as listed in Table 3, for 0.5 seconds per bottle cap from a distance 30 mm upstream side, and the irradiated bottle cap was collected on a sterile petri dish. Here, the operating conditions of the sterilization apparatus were the same as in Test Example B-1. The chamber environment in which the sterilization apparatus was set was such that the temperature was controlled with a heater, and an absolute humidity was set as listed in Table 3.

### Operating Conditions of Sterilization Apparatus

Inlet unit B-1 for alternating current: frequency: 14 kHz, voltage: 300 V, electric current: 11A
High-voltage unit B-2: The raised voltage: 20 kV
Inlet unit B-3 for gas flow: flow rate of air: 6 L/min, flow rate of oxygen: 3 L/min (hereinabove, go to nozzle B-4), flow rate of air: 3 L/min (go to inlet unit B-6 for steam flow)
Nozzle B-4: temperature of reactive oxygen irradiation: 51°C, irradiation speed: 50,000 mm/sec
Chilling unit B-5: chilling water: 5°C
Inlet unit B-6 for steam flow: electric heating wires: 300°C, flow rate of water-containing gas: 4.5 L/min (flow rates of plasma jet/water-containing gas flow (volume ratio) = 9/4.5)
Inlet unit B-7 for water flow: flow rate of water: 0.2 mL/min

### Measurement of Sterilization Activity Values

A resin bottle cap was taken out of the sterile petri dish, and 5 mL of TSA liquid medium (manufactured by BD Falcon) was injected to the bottle cap, and cultured at 35°C, a temperature suitable for proliferation of microbes for 3 days. After the cultivation, the number of bottle caps in which media became turbid due to microbial proliferation that was judged as positive was counted, and the sterilization activity value LRV (Log Reduction Value) was calculated according to the most probable number method (MPN method). The results are shown in Table 2. Here, the "D" value showing the sterilization activity is expressed by common logarithm (LOG value) of the number of bacteria per bottle cap, which is a value obtained by subtracting the number of bacteria after the treatment (LOG value) from the number of bacteria before the treatment (LOG value). It is shown that the larger the number, the higher the sterilization activity, and the number of 4.5 D or more would show no problem as the sterilization treatment of food containers.

### Table 3

**Table 3**

| | Temperature Inside Chamber (°C) | Absolute Humidity (g/m³) | LRV (D Value) |
|---|---|---|---|
| Ex. C-1 | 25 | 15.0 | 4.9 D |
| Ex. C-2 | 40 | 14.6 | 5.0 D |
| Comp. Ex. C-1 | 60 | 14.0 | 3.1 D or Less |

It is suggested from Table 3 that when the conditions of an absolute humidity of from 14.1 to 25.0 g/m³ are satisfied, especially excellent sterilization effects are obtained.

### INDUSTRIAL APPLICABILITY

The sterilization method of the present invention shows excellent sterilization activity, so that it can be suitably used in, for example, sterilization of containers for foodstuff, bottle caps sealing the openings of the containers, medical devices, foodstuff such as vegetables and meat, and the like.

### EXPLANATION OF NUMERALS

- A-1: inlet unit for alternating current
- A-2: high-voltage unit
- A-3: inlet unit for gas flow
- A-4: nozzle
- A-5: chilling unit
- A-6: inlet unit for steam flow
- A-7: inlet unit for water flow
- A-8: inlet unit for oxidizing gas flow
- A-9: chamber
- B-1: inlet unit for alternating current
- B-2: high-voltage unit
- B-3: inlet unit for gas flow
- B-4: nozzle
- B-5: chilling unit
- B-6: inlet unit for steam flow
- B-7: inlet unit for water flow
- B-8: irradiation platform

## Claims

1. Sterilization apparatus comprising:
(i) a chamber (A-9) containing:
(ii) a reactive oxygen irradiation unit (A-4) for irradiating reactive oxygen, wherein the reactive oxygen irradiation unit (A-4) has a nozzle for generating plasma from a carrier gas using alternating current, the plasma being used to generate reactive oxygen, the nozzle having a gas inlet port for the carrier gas and a reactive oxygen irradiation port, at opposite ends thereof;
**characterised in that** the apparatus further comprises:
(iii) a chamber inlet unit (A-8) for oxidizing gas flow, for flowing an oxidizing gas into the chamber, existing in a separate space from the nozzle of the reactive oxygen irradiation unit (A-4);
wherein the apparatus is adapted so that the reactive oxygen is irradiated from the reactive oxygen irradiation unit (A-4) onto an object to be sterilized in the presence of the oxidizing gas.

2. Sterilization apparatus according to claim 1 comprising a gas flow inlet unit (A-3) to flow carrier gas to the nozzle of the reactive oxygen irradiation unit (A-4).

3. Sterilization apparatus according to claim 1 or 2 comprising a steam flow inlet unit (A-6) to flow water-containing gas to the reactive oxygen irradiation port of the nozzle of the reactive oxygen irradiation unit (A-4).

4. Sterilization apparatus according to any one of the preceding claims comprising a chamber (A-9) in which an object to be sterilized can be irradiated with reactive oxygen from the reactive oxygen irradiation unit (A-4), in the presence of the oxidizing gas from the inlet unit (A-8) for oxidizing gas flow.

5. Sterilization apparatus according to any one of the preceding claims wherein the inlet unit (A-8) for oxidizing gas flow comprises means to provide hydrogen peroxide or peracetic acid as said oxidizing gas.

6. A sterilization method comprising irradiating reactive oxygen onto an object to be sterilized **characterised in that** the irradiating occurs in the presence of an oxidizing gas, in sterilization apparatus according to any one of claims 1 to 5.

7. A sterilization method according to claim 6 wherein the oxidizing gas is selected from hydrogen peroxide and peracetic acid.

8. A sterilization method according to claim 6 or 7 wherein the amount of the oxidizing gas is 0.1 ppmv or more and/or 2 ppmv or less.

9. A sterilization method according to claim 6 or 7 in which the reactive oxygen is irradiated onto the object under an environment of from 50 to 100% relative humidity.

10. A sterilization method according to any one of claims 6 to 9 in which the object to be sterilized is selected from containers for foodstuff, bottle caps for sealing the opening parts of containers, medical devices and foodstuffs such as vegetables and meat.

## Patentansprüche

1. Sterilisierungsvorrichtung, die Folgendes umfasst:
(i) eine Kammer (A-9), die Folgendes enthält:
(ii) eine Reaktiver-Sauerstoff-Bestrahlungseinheit (A-4) zum Bestrahlen durch reaktiven Sauerstoff, wobei die Reaktiver-Sauerstoff-Bestrahlungseinheit (A-4) eine Düse zum Erzeugen von Plasma aus einem Trägergas unter Verwendung von Wechselstrom aufweist, wobei das Plasma verwendet wird, um reaktiven Sauerstoff zu erzeugen, wobei die Düse an ihren einander entgegengesetzten Enden eine Gaseinlassöffnung für das Trägergas und eine Reaktiver-Sauerstoff-Bestrahlungsöffnung umfasst;
**dadurch gekennzeichnet, dass** die Vorrichtung außerdem Folgendes umfasst:
(iii) eine Kammereinlasseinheit (A-8) für das Einströmen von oxidierendem Gas, um ein oxidierendes Gas in die Kammer strömen zu lassen, die sich in einem separaten Raum von der Düse der Reaktiver-Sauerstoff-Bestrahlungseinheit (A-4) befindet;
wobei die Vorrichtung so ausgelegt ist, dass der reaktive Sauerstoff von der Reaktiver-Sauerstoff-Bestrahlungseinheit (A-4) auf ein zu sterilisierendes Objekt in Gegenwart des oxidierenden Gases gestrahlt wird.

2. Sterilisierungsvorrichtung nach Anspruch 1, die eine Gasströmungs-Einlasseinheit (A-3) umfasst, um Trägergas zu der Düse der Reaktiver-Sauerstoff-Bestrahlungseinheit (A-4) strömen zu lassen.

3. Sterilisierungsvorrichtung nach Anspruch 1 oder 2, die eine Dampfströmungs-Einlasseinheit (A-6) umfasst, um wasserhältiges Gas zu der Reaktiver-Sauerstoff-Bestrahlungsöffnung der Düse der Reaktiver-Sauerstoff-Bestrahlungseinheit (A-4) strömen zu lassen.

4. Sterilisierungsvorrichtung nach einem der vorangegangenen Ansprüche, die eine Kammer (A-9) umfasst, in der ein zu sterilisierendes Objekt mit reaktivem Sauerstoff von der Reaktiver-Sauerstoff-Bestrahlungseinheit (A-4), in Gegenwart des oxidierenden Gases von der Einlasseinheit (A-8) für das Einströmen von oxidierendem Gas, bestrahlt werden kann.

5. Sterilisierungsvorrichtung nach einem der vorangegangenen Ansprüche, wobei die Einlasseinheit (A-8) für das Einströmen von oxidierendem Gas Mittel umfasst, um Wasserstoffperoxid oder Peroxyessigsäure als das oxidierende Gas bereitzustellen.

6. Sterilisierungsverfahren, das das Bestrahlen eines zu sterilisierenden Objekts mit reaktivem Sauerstoff umfasst, **dadurch gekennzeichnet, dass** das Bestrahlen in Gegenwart eines oxidierenden Gases, in einer Sterilisierungsvorrichtung nach einem der Ansprüche 1 bis 5, erfolgt.

7. Sterilisierungsverfahren nach Anspruch 6, wobei das oxidierende Gas aus Wasserstoffperoxid und Peroxyessigsäure ausgewählt ist.

8. Sterilisierungsverfahren nach Anspruch 6 oder 7, wobei die Menge des oxidierenden Gases 0,1 ppmv oder mehr und/oder 2 ppmv oder weniger beträgt.

9. Sterilisierungsverfahren nach Anspruch 6 oder 7, wobei der reaktive Sauerstoff in einer Umgebung von 50 bis 100 % relativer Feuchtigkeit auf das Objekt gestrahlt wird.

10. Sterilisierungsverfahren nach einem der Ansprüche 6 bis 9, wobei das zu sterilisierende Objekt aus Behältern für Lebensmittel, Flaschenverschlüssen zum Verschließen von Öffnungsteilen von Behältern, medizinischen Vorrichtungen und Lebensmitteln wie Gemüse und Fleisch ausgewählt ist.

## Revendications

1. Appareil de stérilisation comprenant :
(i) une chambre (A-9) contenant :
(ii) une unité d'irradiation d'oxygène réactif (A-4) pour irradier de l'oxygène réactif,
dans lequel l'unité d'irradiation d'oxygène réactif (A-4) a une buse pour générer du plasma à partir d'un gaz porteur en utilisant un courant alternatif, le plasma étant utilisé pour générer de l'oxygène réactif, la buse ayant un orifice d'entrée de gaz pour le gaz porteur et un orifice d'irradiation d'oxygène réactif, à des extrémités opposées de celui-ci ;
**caractérisé en ce que** l'appareil comporte en outre :
(iii) une unité d'entrée de chambre (A-8) pour un écoulement de gaz oxydant, pour provoquer un écoulement d'un gaz oxydant jusque dans la chambre, existant dans un espace séparé de la buse de l'unité d'irradiation d'oxygène réactif (A-4) ;
dans lequel l'appareil est adapté de sorte que l'oxygène réactif est irradié à partir de l'unité d'irradiation d'oxygène réactif (A-4) jusque sur un objet à stériliser en présence du gaz oxydant.

2. Appareil de stérilisation selon la revendication 1, comprenant une unité d'entrée d'écoulement de gaz (A-3) pour provoquer un écoulement de gaz porteur vers la buse de l'unité d'irradiation d'oxygène réactif (A-4).

3. Appareil de stérilisation selon la revendication 1 ou 2, comprenant une unité d'entrée d'écoulement de vapeur (A-6) pour provoquer un écoulement de gaz contenant de l'eau vers l'orifice d'irradiation d'oxygène réactif de la buse de l'unité d'irradiation d'oxygène réactif (A-4).

4. Appareil de stérilisation selon l'une quelconque des revendications précédentes, comprenant une chambre (A-9) dans laquelle un objet à stériliser peut être irradié avec de l'oxygène réactif provenant de l'unité d'irradiation d'oxygène réactif (A-4), en présence du gaz oxydant provenant de l'unité d'entrée (A-8) pour un écoulement de gaz oxydant.

5. Appareil de stérilisation selon l'une quelconque des revendications précédentes, dans lequel l'unité d'entrée (A-8) pour un écoulement de gaz oxydant comprend des moyens pour fournir du peroxyde d'hydrogène ou de l'acide peracétique en tant que gaz oxydant.

6. Procédé de stérilisation comprenant l'irradiation d'oxygène réactif jusque sur un objet à stériliser, **caractérisé en ce que** l'irradiation survient en présence d'un gaz oxydant, dans un appareil de stérilisation selon l'une quelconque des revendications 1 à 5.

7. Procédé de stérilisation selon la revendication 6, dans lequel le gaz oxydant est choisi parmi le peroxyde d'hydrogène et l'acide peracétique.

8. Procédé de stérilisation selon la revendication 6 ou 7, dans lequel la quantité de gaz oxydant est de 0,1 ppmv ou plus et/ou de 2 ppmv ou moins.

9. Procédé de stérilisation selon la revendication 6 ou 7, dans lequel l'oxygène réactif est irradié jusque sur l'objet dans un environnement d'humidité relative de 50 à 100 %.

10. Procédé de stérilisation selon l'une quelconque des revendications 6 à 9, dans lequel l'objet à stériliser est choisi parmi des contenants de produit alimentaire, des bouchons de bouteille pour sceller les parties d'ouverture de contenants, des instruments médicaux et des produits alimentaires tels que des légumes et de la viande.
